# EUROPEAN PATENT SPECIFICATION

(11) **EP 3 039 017 B1**
(45) Date of publication and mention of the grant of the patent: **09.01.2019**
(21) Application number: 14755816.7
(22) Date of filing: 26.08.2014
(51) Int. Cl.: C07D 311/70, C07D 311/72

(54) **FORMATION OF CHROMANES AND CHROMENES BY USING SILVER(I) OR GOLD(I) SALTS OR COMPLEXES**
BILDUNG VON CHROMANEN UND CHROMENEN DURCH VERWENDUNG VON SILBER(I)- ODER GOLD(I)-SALZEN ODER -KOMPLEXEN
FORMATION DE CHROMANES ET CHROMÈNES EN UTILISANT DES SELS OU DES COMPLEXES D'ARGENT (I) OU D'OR (I)

(30) Priority: 30.08.2013 EP 13182427
(43) Date of publication of application: 06.07.2016
(73) Proprietor: DSM IP Assets B.V., 6411 TE Heerlen (NL)
(72) Inventor: LETINOIS, Ulla, CH-4002 Basel (CH); NETSCHER, Thomas, CH-4002 Basel (CH); STEMMLER, René Tobias, CH-4002 Basel (CH)
(74) Representative: Dux, Roland
(86) International application number: PCT/EP2014/068080
(87) International publication number: WO 2015/028463

(56) References cited:
- URS HENGARTNER ET AL: "Asymmetric Synthesis of [alpha]-Tocopherol", CHEMISTRY - A EUROPEAN JOURNAL, vol. 16, no. 4, 25 January 2010 (2010-01-25), pages 1306-1311, XP055032731, ISSN: 0947-6539, DOI: 10.1002/chem.200902754
- IOANNIS N. LYKAKIS ET AL: "Ph3PAuNTf2 as a Superior Catalyst for the Selective Synthesis of 2H-Chromenes: Application to the Concise Synthesis of Benzopyran Natural Products", EUROPEAN JOURNAL OF ORGANIC CHEMISTRY, vol. 2011, no. 12, 10 April 2011 (2011-04-10), pages 2334-2338, XP055082767, ISSN: 1434-193X, DOI: 10.1002/ejoc.201001674
- NICOLAS MÉZAILLES ET AL: "Phosphine Gold(I) Bis-(trifluoromethanesulfonyl)imidate Complexes as New Highly Efficient and Air-Stable Catalysts for the Cycloisomerization of Enynes", ORGANIC LETTERS, vol. 7, no. 19, 1 September 2005 (2005-09-01), pages 4133-4136, XP055083124, ISSN: 1523-7060, DOI: 10.1021/ol0515917
- NEVADO C ET AL: "Intramolecular Hydroarylation of Alkynes Catalyzed by Platinum or Gold: Mechanism and endo Selectivity", CHEMISTRY - A EUROPEAN JOURNAL, WILEY - V C H VERLAG GMBH & CO. KGAA, WEINHEIM, DE, vol. 11, no. 10, 6 May 2005 (2005-05-06), pages 3155-3164, XP002557136, ISSN: 0947-6539, DOI: 10.1002/CHEM.200401069 [retrieved on 2005-03-18]
- HUO ET AL: "Enantioselective total synthesis of hydramicromelin B", TETRAHEDRON ASYMMETRY, PERGAMON PRESS LTD, OXFORD, GB, vol. 19, no. 3, 7 February 2008 (2008-02-07), pages 343-347, XP022479395, ISSN: 0957-4166, DOI: 10.1016/J.TETASY.2008.01.006
- MARGARET A. BRIMBLE ET AL: "Synthesis of the Tetracyclic Core of Berkelic Acid Using Gold(I)-Catalyzed Hydroarylation and Oxidative Radical Cyclizations", ORGANIC LETTERS, vol. 14, no. 23, 7 December 2012 (2012-12-07), pages 5820-5823, XP055082773, ISSN: 1523-7060, DOI: 10.1021/ol302536j
- URSULA KOCH-POMERANZ ET AL: "Die durch Silberionen katalysierte Umlagerung von Propargyl-phenylther", HELVETICA CHIMICA ACTA, vol. 56, no. 8, 1 January 1973 (1973-01-01), pages 2981-3004, XP055083162,

## Description

### Technical Field

The present invention relates to the field of the synthesis of chromanes and chromenes, particularly, of (3,4)-dehydrotocopherols, (3,4)-dehydrotocotrienols, tocopherols and tocotrienols.

### Background of the invention

An important class of chromane compounds are vitamin E and its esters. Often vitamin E is commercialized in the form of its esters because the latter show an enhanced stability, particularly against humidity, air and oxidating agents.

On the one hand the typical technical synthesis of vitamin E leads to mixtures of isomers. On the other hand higher bioactivity (biopotency) has been shown to occur in general by tocopherols and tocotrienols having the R-configuration at the chiral centre situated next to the ether atom in the ring of the molecule (indicated by * in the formulas used later on in the present document) (i.e. 2R-configuration), as compared to the corresponding isomers having S-configuration. Particularly active are the isomers of tocopherols having the natural configuration at all chiral centres, for example (R,R,R)-tocopherols, as has been disclosed for example by H. Weiser et al. in J. Nutr. 1996, 126(10), 2539-49. This leads to a strong desire for an efficient process for separating the isomers. Hence, the isomer separation not only of vitamin E, but also of their esters, particularly their acetates, as well as of their precursors is of prime interest.

Separation of all the isomers of tocopherols by chromatographic methods is extremely difficult and costly.

To overcome these inherent problems, it has been tried to offer stereospecific synthesis allowing the preferential formation of the desired isomers only. However, these methods are very expensive, complex and/or exotic as compared to the traditional industrial synthesis leading to isomer mixtures.

Therefore, there exists a large interest in providing stereospecific synthesis routes leading to the desired isomer.

Particular difficult is to achieve specifically the desired chirality at the chiral carbon centre in the 2-position of the chromane ring by chemical synthesis.

A synthetic pathway for chromanes is via their corresponding chromenes. The hydroarylation of aryl propargyl ethers leads to chromenes. In most cases this reaction is conducted thermally, often in the high boiling solvent diethyl aniline.

Hydroarylations of aryl propargyl ethers can be carried out upon thermal activation (150-200 °C). The disadvantage of thermally activated hydroarylations is the need for high temperatures, as thermally unstable compounds cannot be used for this transformation.

Transition metals such as Re, Pd, and Pt have been used to catalyse the hydroarylation of alkynes.

It has been found that also Ag(I) and Au(I) catalyse the hydroarylation. Reetz M.T. et al. in Eur. J. Org. Chem. 2003, 3485-3496 disclose that Au(I) catalyses the intermolecular reaction of alkynes with arenes which can be activated by addition of Ag(I).

Nevado C. et al. disclose in Chem. Eur. J. 2005, 11, 3155-3164 an intramolecular hydroarylation of alkynes using gold(I) complexes. It further discloses that this reaction leads to significant amounts of by-products having either a 5-membered ring or a different substitution pattern or different position of the double bond. Furthermore, it mentions that the use of Ag(I) leads to unexpected dimerization of the desired chromene.

U. Hengartner et al., Chem. Eur. J. 2010, 16, 1306-1311 discloses in a attempted Mitsunobu rection of monoprotected hydroquinones with tertiary alcohols (scheme 2).

I.N. Lykakis et al., Eur. J. Org. Chem. 2011, 2334-2338 discloses the cycloisomerization of aryl propargyl ethers into 2*H*-chromenes and 2-methylbenzofurans by gold catalysts.

However, none of the documents deals with the formation of chiral chromene entities of the desired structure by using hydroarylation at low temperatures and with high yield.

### Summary of the invention

Therefore, the problem to be solved by the present invention is to offer a method for the synthesis of chiral chromenes or chromanes, i.e. of compounds of formula (I) or (IV) in a stereospecific manner in view of the 2 position in the chromene or chromane ring.

Surprisingly, it has been found that a process for the manufacturing according to claim 1 or claim 9 is able to solve this problem.

It has been shown that this process can be carried out at very low temperature, i.e. below 40°C, and that the configuration at the chiral centre is not influenced or altered by the reaction steps.

The process shows high yields and high selectivity in the desired product.

It further has been observed that no by-product, such as the formation of products having 5-rings, or by alternate ring closure leading to different substitution pattern at the chromene, or by dimerization of chromenes, have been found.

Therefore, this method is ideally suited for the chemical synthesis of chiral chromenes or chromanes, respectively, leading to or found in vitamin E.

Further aspects of the invention are subject of further independent claims. Particularly preferred embodiments are subject of dependent claims.

### Detailed description of the invention

In a first aspect the present invention relates to a method of preparing compound of formula (I) according to claim 1. It comprises the steps 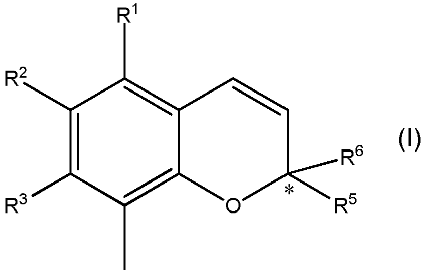
a) providing compound of formula (II) 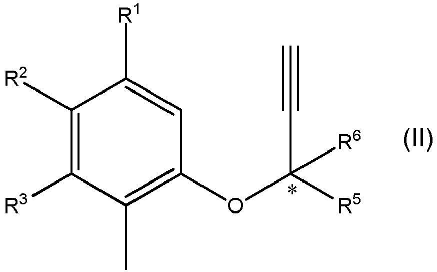
b) adding
   at least one silver(I) or gold(I) salt or complex and
   at least one acid (**A**) which is selected from the group consisting of BX₃, the adducts of BX₃ with alcohols or ethers, HBX₄, H[PX₆], H[SbF₆], HClO₄, H₂SO₄, trifluoroacetic acid, sulfonic acids and sulfonimides of formula (HAN-1); 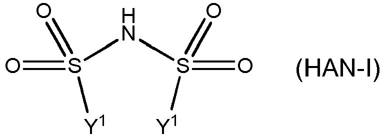 and/or
   at least one silver(I) or gold(I) metal salt or complex having an anion (**AN**) which is selected from the group consisting of [BX₄]⁻, [PX₆]⁻, [SbF₆]⁻, [ClO₄]⁻, CF₃COO⁻, sulfonates, tetra(3,5-bis(trifluoromethyl)phenyl)borate (BAr_{F}⁻), tetraphenylborate, and anions of formula (AN-1); 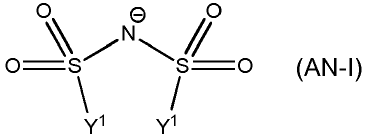 wherein X represents a halogen atom;
   and Y¹ represents a phenyl or a C₁₋₈-alkyl group which preferably is substituted by at least one halogen atom to compound of formula (II) forming a mixture;
c) submitting said mixture to a temperature in the range between -15°C and 40°C, preferably between 15 °C and 40°C;
   wherein R¹ and R³ are independently from each other hydrogen or methyl groups;
   R² represents either hydrogen or a methyl group or a group OR^{2'} where R^{2'} represents hydrogen or a phenol protection group;
   R⁵ represents either a completely saturated C₁₋₂₅-alkyl group or a C₁₋₂₅-alkyl group comprising at least one carbon-carbon double bond;
   R⁶ represents either H or a C₁₋₂₅-alkyl group, particularly a methyl group; with the proviso that R⁵ is different from R⁶;
   and wherein each * represents a chiral centre and this chiral centre has the same configuration in formula (I) and formula (II).
By having different substituents R⁵ and R⁶ in formula (I) and (II), the carbon centre attached to said groups, i.e. the carbon centre indicated by *, is chiral.

The term "independently from each other" in this document means, in the context of substituents, moieties, or groups, that identically designated substituents, moieties, or groups can occur simultaneously with a different meaning in the same molecule.

The term "vitamin E" is used in the present document as a generic descriptor for all tocol and tocotrienol derivatives exhibiting qualitatively the biological activity of α-tocopherol (IUPAC-IUB Recommendation 1981, Eur. J. Biochem. 123, 473-475 (1982)).

A "C_{x-y}-alkyl" group is an alkyl group comprising x to y carbon atoms, i.e., for example, a C₁₋₃-alkyl group is an alkyl group comprising 1 to 3 carbon atoms. The alkyl group can be linear or branched. For example -CH(CH₃)-CH₂-CH₃ is considered as a C₄-alkyl group.

The term "hydrogen" means in the present document H and not H₂. The term "molecular hydrogen" means H₂.

The signs * and # in formulae of molecules represent in this document a chiral centre in said molecule.

In the present document any single dotted line represents the bond by which a substituent is bound to the rest of a molecule.

The chirality of an individual chiral carbon centre is indicated by the label R or S according to the rules defined by R. S. Cahn, C. K. Ingold and V. Prelog. This R/S-concept and rules for the determination of the absolute configuration in stereochemistry is known to the person skilled in the art.

Substance names starting with "poly" such as polycarboxylic acid as used in the present document refer to substances formally containing two or more of the corresponding functional groups per molecule.

In case identical labels for symbols or groups are present in several formulae, in the present document, the definition of said group or symbol made in the context of one specific formula applies also to other formulae which comprises said same label.

The expression "process of preparation" is a synonym for "method of preparation" and can be used interchangeable to each other.

The anion tetra(3,5-bis(trifluoromethyl)phenyl)borate is abbreviated in the present document as "BA_{RF}⁻" being known to the person skilled in the art also by the abbreviation "[BAr^{F}₄]⁻".

The term "propargylic aryl ethers" as used in this document is an ether of the following formula 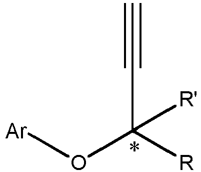 in which Ar represents an aromatic ring which might be further substituted and R and R' each represent an hydrogen atom or any alkyl group which might be saturated or unsaturated. Therefore, a propargylic aryl ether in the sense of the invention has a key feature that the oxygen ether atom is directly linked to an aromatic system. In the case where R is different from R' the carbon centre between the ether oxygen and the carbon-carbon triple bond is chiral, the configuration of which is characterized by having the S- or the R-configuration. To a propargylic aryl ether of this type is referred when the term "chiral propargylic aryl ether" is used in this document.

In the first step (step a)) of the method (or process) of preparing the compound of formula (I), compound of formula (II) is provided.

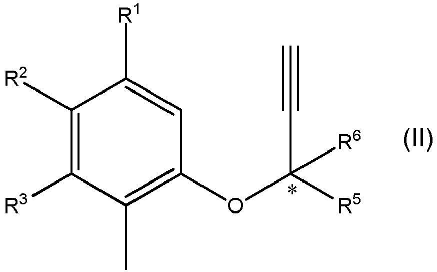

R⁶ represents either H or a C₁₋₂₅-alkyl group, particularly a methyl group.

In a preferred embodiment R⁶ represents a methyl group.

As R⁵ is different from R⁶ the compound of formula (II) is chiral.

In a preferred embodiment R⁵ is of formula (III).

In a more preferred embodiment R⁶ represents a methyl group and R⁵ is of formula (III)

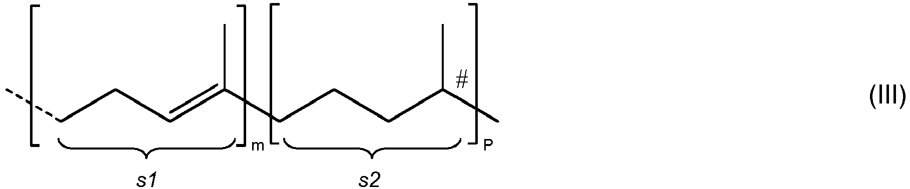

In formula (III) m and p stand independently from each other for a value of 0 to 5 provided that the sum of m and p is 1 to 5. Furthermore, the substructures in formula (III) represented by *s1* and *s2* can be in any sequence. The dotted line represents the bond by which the substituent of formula (III) is bound to the rest of the compound of formula (II-B) or formula (I). Furthermore, # represents a chiral centre, obviously except in case where said centre is linked to two methyl groups.

It is preferred that group R⁵ is of formula (III-x).

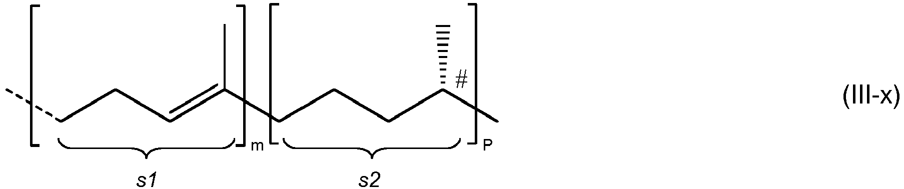

As mentioned above the substructures in formula (III) represented by *s1* and *s2* can be in any sequence. It is, therefore, obvious that in case that the terminal group is having the substructure *s2,* this terminal substructure has no chiral centre.

In one preferred embodiment m stands for 3 and p for 0.

In another preferred embodiment p stands for 3 and m for 0.

Therefore, R⁵ is preferably of formula (III-A), particularly (III-ARR), or (III-B).

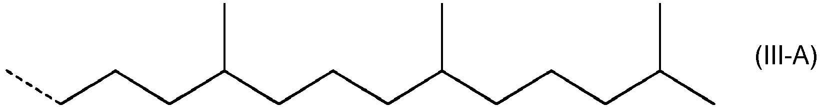

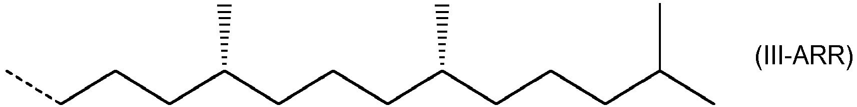

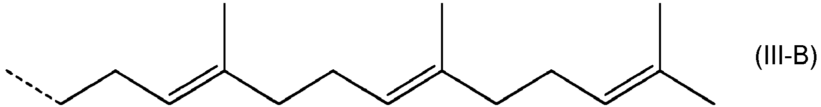

Preferred are the following combinations of R¹ and R³:
R¹ = R³ = CH₃
   or
R¹ = CH₃, R³ = H
   or
R¹ = H, R³ = CH₃
   or
R¹ = R³ = H

Most preferred is R¹ = R³ = CH₃.

R² represents either hydrogen or a methyl group or a group OR^{2'} where R^{2'} represents hydrogen or a phenol protection group.

In one preferred embodiment R² represents a group OR^{2'} where R^{2'} represents hydrogen or a phenol protection group.

In other words, in one of the most preferred embodiments R² stands for OH.

In another most preferred embodiment, a phenol protecting group R^{2'} is linked to the oxygen atom which is directly linked to the aromatic ring.

A phenol protection group is a group which protects the phenolic group (R²=OH in formula (I) or (II)) and can be deprotected easily, i.e. by state-of-the-art methods, to the phenolic group again.

The phenol protection group forms with the rest of the molecule a chemical functionality which is particularly selected from the group consisting of ester, ether or acetal. The protection group can be easily removed by standard methods known to the person skilled in the art.

In case where the phenol protection group forms with the rest of the molecule an ether, the substituent R^{2'} is particularly a linear or branched C₁₋₁₀-alkyl or cycloalkyl or aralkyl group. Preferably the substituent R^{2'} is a benzyl group or a substituted benzyl group, particularly preferred a benzyl group.

In case where the phenol protection group forms with the rest of the molecule an ester, the ester is an ester of an organic or inorganic acid.

If the ester is an ester of an organic acid, the organic acid can be a monocarboxylic acid or a polycarboxylic acid, i.e. an acid having two or more COOH-groups. Polycarboxylic acid are preferably malonic acid, succinic acid, glutaric acid, adipic acid, maleic acid or fumaric acid.

Preferably the organic acid is a monocarboxylic acid.

Hence, the substituent R^{2'} is preferably an acyl group. The acyl group is particularly a C₁₋₇-acyl, preferably acetyl, trifluoroacetyl, propionyl or benzoyl group, or a substituted benzoyl group.

If the ester is an ester of an inorganic acid, the inorganic acid is preferably nitric acid or a polyprotic acid, i.e. an acid able to donate more than one proton per acid molecule, particularly selected from the group consisting of phosphoric acid, pyrophosphoric acid, phosphorous acid, sulphuric acid and sulphurous acid.

In case where the phenol protection group forms with the rest of the molecule an acetal, the substituent R^{2'} is preferably 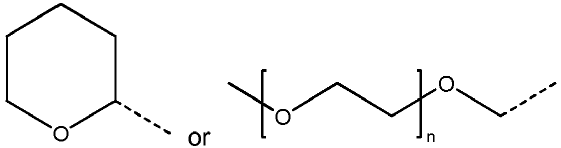 with n=0 or 1.

Hence, the acetals formed so are preferably methoxymethyl ether (MOM-ether), β-methoxyethoxymethyl ether (MEM-ether) or tetrahydropyranyl ether (THP-ether). The protection group can easily be removed by acid.

The protecting group is introduced by reaction of the corresponding molecule having an R^{2'} being H (corresponding to R²=OH) with a protecting agent.

The protecting agents leading to the corresponding phenol protection groups are known to the person skilled in the art, as well as the chemical process and conditions for this reaction. If, for example, the phenol protection group forms with the rest of the molecule an ester, the suitable protecting agent is for example an acid, an anhydride or an acyl halide.

In the case that an ester is formed by the above reaction with the protecting agent, and that said ester is an ester of an organic polycarboxylic acid or an inorganic polyprotic acid, not necessarily all acid groups are esterified to qualify as protected in the sense of this document. Preferable esters of inorganic polyprotic acids are phosphates.

It is preferred that the protection group R^{2'} is a benzoyl group or a C₁₋₄-acyl group, particularly acetyl group or trifluoroacetyl. The molecules in which R^{2'} represents an acyl group, particularly an acetyl group, can be easily prepared from the corresponding unprotected molecule by esterification, respectively the phenolic compound can be obtained from the corresponding ester by ester hydrolysis.

The phenol protecting group is preferably selected such that it is cleaved inside the human or animal body, particularly in the stomach.

It is important to realize that the step of reacting with the protecting agent can occur at different stages of manufacture of compound of formula (I) or of formula (II), i.e. the reaction with the protecting agent can occur for example before or after preparation of compound (I) or compound (II).

Compound of formula (II) is obtained by the following reaction scheme:

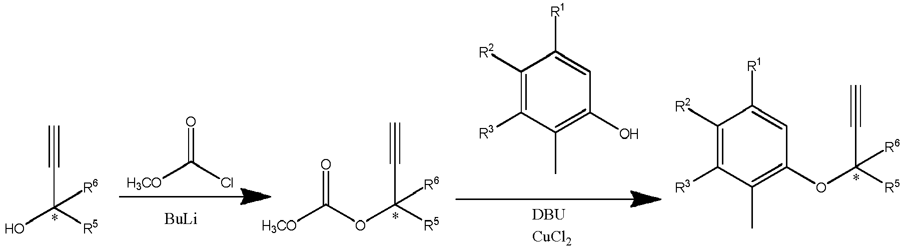

The required chiral quaternary aryl propargyl ethers are available by synthesis as described in literature, for example using lithium acetylides catalysed by lithium binaphtholate or diethyl zinc in the presence of ephedrine-based ligands optionally followed by suitable purification methods.

In one embodiment in the second step (b)) of the method of preparing the compound of formula (I) at least one silver(I) or gold(I) salt or complex and at least one acid (**A**) which is selected from the group consisting of BX₃, the adducts of BX₃ with alcohols or ethers, HBX₄, H[PX₆], H[SbF₆], HClO₄, H₂SO₄, trifluoroacetic acid, sulfonic acids and sulfonimides of formula (HAN-1) is added to the compound of formula (II) being provided in step a) to form a mixture.

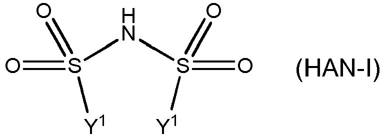

Y¹ represents a phenyl or a C₁₋₈-alkylgroup which preferably is substituted by at least one halogen atom.

Preferably Y¹ represents a CF₃ group. So a preferable sulfonimides of formula (HAN-1) is bis(trifluoromethane)sulfonimide, which is also known as triflimidic acid.

Preferred sulfonic acids are halogenated sulfonic acids, particularly trifluoromethanesulfonic acid, which is also known as triflic acid.

In one of the preferred embodiments the acid (**A**) is BX₃, particularly BF₃, respectively the adducts thereof with alcohols or ethers, particularly BF₃-dialkylether adducts.

In a more preferred embodiment the acid (**A**) in step b) is an acid which is selected from the group consisting of BF₃, HBF₄, triflic acid and triflimidic acid.

It is preferred that in step b) the silver (I) or gold(I) salt or complex is a chloro complex of Au(I) and the acid (**A**) is BF₃, respectively the adducts thereof with alcohols or ethers, particularly BF₃-dialkylether adducts.

The molar ratio of silver(I) or gold(I) salt or complex to compound of formula (II) is preferably between 4 and 0.01 mol-%, particularly between 2 and 0.3 mol-%.

The molar ratio of acid (**A**) to silver(I) or gold(I) salt or complex is preferably between 300 and 100 mol-%, particularly between to 150 and 100 mol-%.

In a second embodiment in the second step (b)) of the method of preparing the compound of formula (I) at least one silver(I) or gold(I) metal salt or complex having an anion (**AN**) which is selected from the group consisting of [BX₄]⁻, [PX₆]⁻, [SbF₆]⁻, [ClO₄]⁻, CF₃COO⁻, sulfonates, tetra(3,5-bis(trifluoromethyl)-phenyl)borate (BAr_{F})⁻, tetraphenylborate, and anions of formula (AN-1); is added to the compound of formula (II) being provided in step a) to form a mixture.

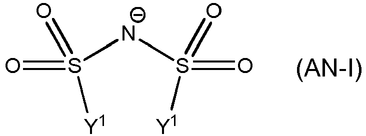

Preferably Y¹ represents a CF₃ group. So preferably the anions of formula sulfonimides of formula (AN-1) is the anion of of bis(trifluoromethane)sulfonimide, which is also known as triflimidic acid.

Preferred sulfonates are halogenated anions of sulfonic acids, particularly of trifluoromethanesulfonic acid, which is also known as triflic acid. Therefore, the preferred sulfonates are trifluoromethanesulfonates, which are also known as triflates.

In a more preferred embodiment the anion (**AN**) in step b) is an anion which is selected from the group consisting of [BX₄]⁻, triflate, and anions of formula (AN-1).

It is preferred that in step b) the silver(I) or gold(I) salt or complex having an anion (**AN**) is silver(I) triflate or silver(I) tetrafluoroborate.

The molar ratio of silver(I) or gold(I) metal salt or complex having an anion (**AN**) to compound of formula (II) is preferably between 4 and 0.01 mol-%, particularly between 2 and 0.3 mol-%.

In a third embodiment in the second step (b)) at least one silver(I) or gold(I) salt or complex and at least one acid (**A**) and at least one silver(I) or gold(I) metal salt or complex having an anion (**AN**) is added to the compound of formula (II) being provided in step a) to form a mixture. The acid (**A**) and the anion (**AN**) are as disclosed above.

It is preferred that in step b) a combination of Ag(I) triflate or Ag(I) tetrafluoroborate and a chloro complex of Au(I) is used.

In case that in step b) a silver(I) or gold(I) complex is used it is preferred that said complex has at least one phosphorous containing ligand, particularly selected from the group consisting of 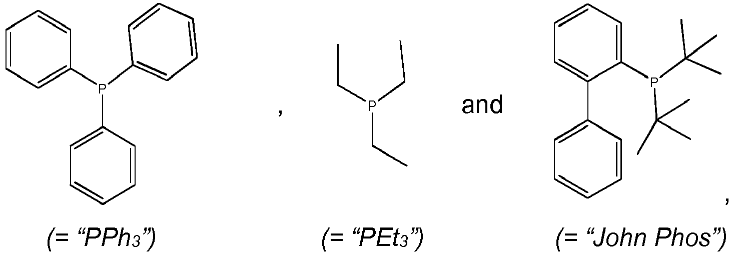 or an imidazole-2-ylidene, particularly 1,3-bis(2,6-diisopropylphenyl)-1,3-dihydro-2*H*-imidazol-2-ylidene ((="[(iPr₂)Ph₂]lm")

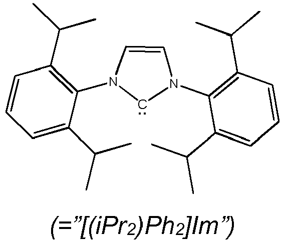

In the third step (step c)) of the method of preparing the compound of formula (I), mixture of step b) is submitted to a temperature in the range between -15°C and 40°C, preferably between 15°C and 30°C.

By this process the intramolecular addition of formula (II) compound of formula (I) is yielded. It is preferred that the addition reaction is made in a solvent. Preferably the solvent is selected from the group consisting of hydrocarbons, halogenated hydrocarbons. More preferably the solvent is toluene or dichloromethane. The solvent is preferably added before or during or after step b).

The present method leads in a high yield to the compound of formula (I). It has been observed that the configuration at the chiral centre * of formula (II) is maintained during the intramolecular reaction. Hence, it is possible to have prepared compounds of formula (I) in a high excess of a specific configuration at the chiral centre indicated by *. Particularly, it is possible to have prepared compounds of formula (I) in a ratio of the R-configuration to the S-configuration at the chiral carbon centre indicated by * of more than 90:10, preferably more than 95:5, particularly more than 98:2. Starting from the other isomer of formula (II), accordingly, it is possible to have prepared compounds of formula (I) in a ratio of the R-configuration to the S-configuration at the chiral carbon centre indicated by * of more than 10:90, preferably more than 5:95, particularly more than 2:98.

Therefore, preferred compound (II) is a compound of formula (II-A).

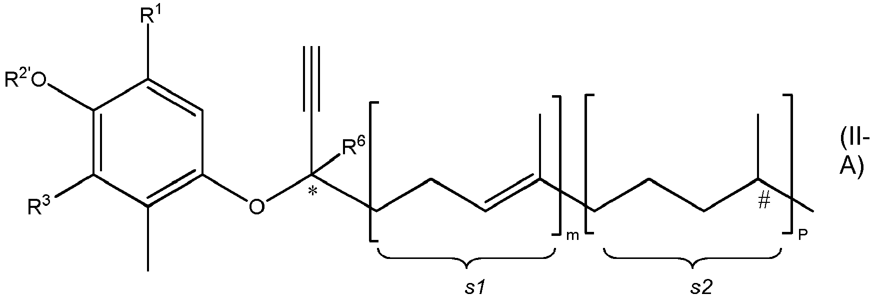

The groups R¹, R³, R^{2'}, R⁶ and *, m, p, *s1* and *s2* as well the preferred embodiments thereof have already discussed in details before for compound of formula (II) in which R⁵ is of formula (III). This compound is a preferred embodiment of the present invention and is particularly useful for the process of the mentioned method.

Particularly, the sum of m and p is 1 to 5.

Particularly, preferred are the compounds of formula (II) are compounds of formula (II-A-1), (II-A-1RR) and (II-A-2).

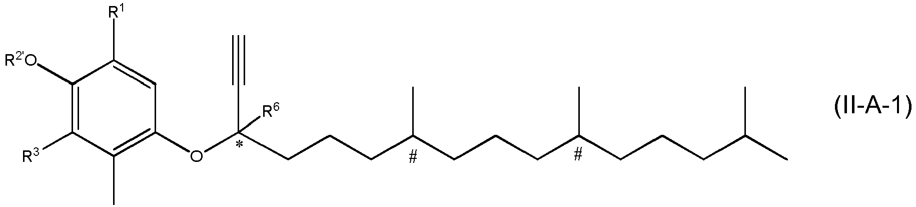

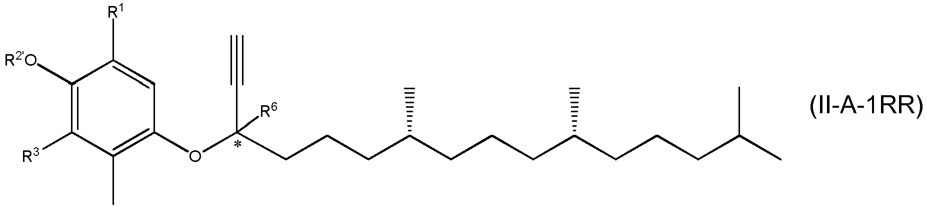

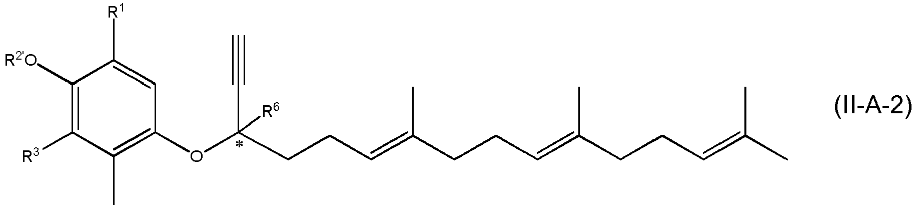

Hence, the preferred compound of formula (I) being formed by the above discussed method is of formula (I-A)

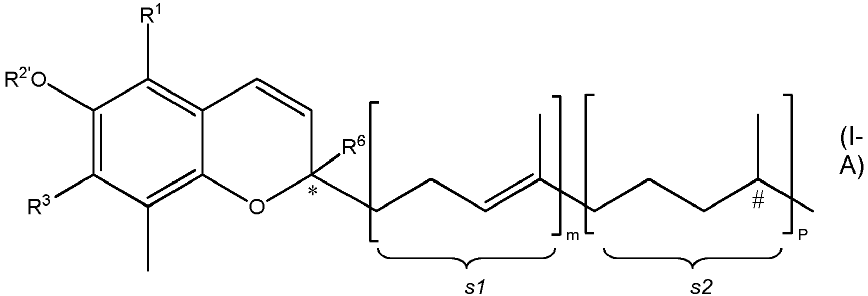

Particularly, preferred are the compounds of formula (I) are compounds of formula (I-A-1), (I-A-1RR) and (I-A-2).

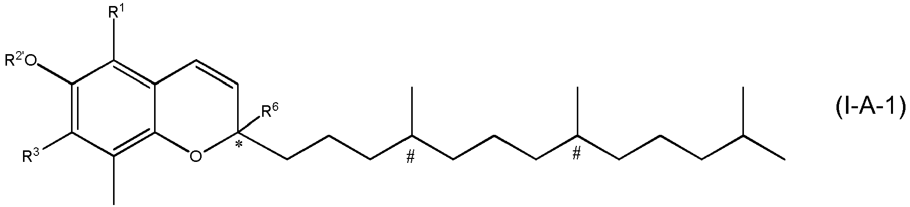

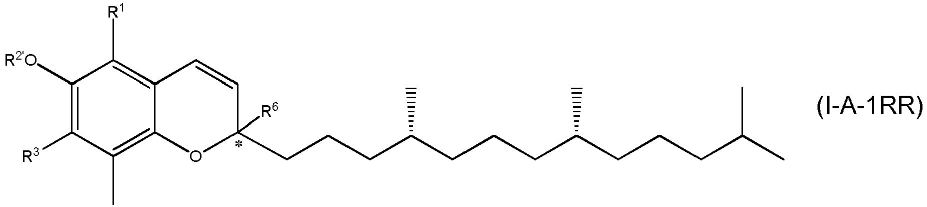

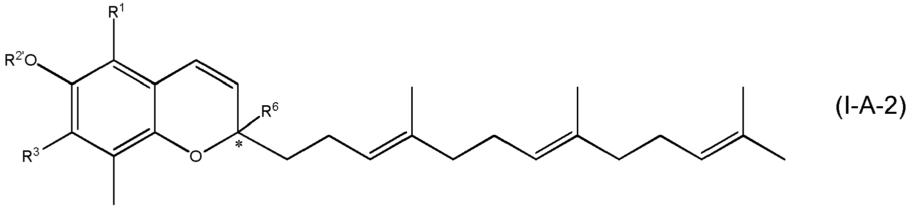

Most preferred are the compounds of formula (I) are compounds of formula (I-A-1RRR) and (I-A-2R).

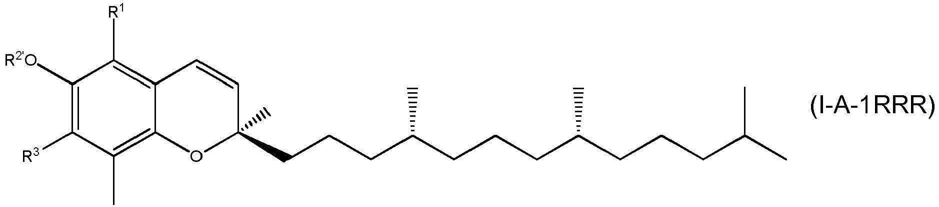

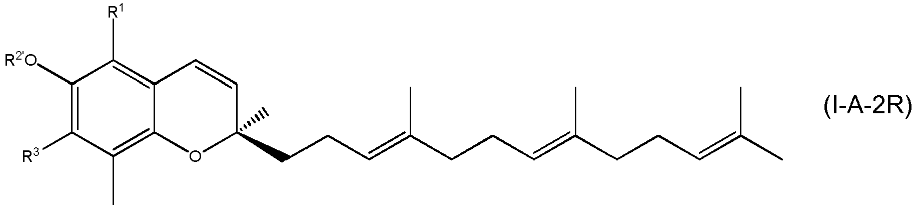

In formulae (II-A), (II-A-1), (I-A) and (I-A-1) # represents a chiral centre, obviously except in case where said centre is linked to two methyl groups. The configuration at said chiral centre in formula (II-A) or (II-A-1) is the same as in formula (I-A) or (I-A-1)

(R,R,R)-dehydroisophythol (= ((3R,7R,11R)-3,7,11,15-tetramethylhexa-dec-1-yn-3-ol)) (formula (VI) is the starting material for the synthesis of formation of compound of formula (I-A-1 RRR). 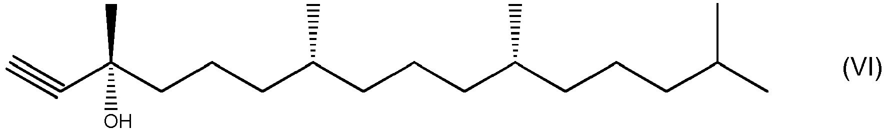 This compound is accessible from natural (E,R,R)-phytol being epoxidized by Sharpless oxidation, converted to the corresponding epoxychloride, followed by multiple lithiation as disclosed in Takano, S.; Sugihara, T.; Ogasawara, K. Synlett 1990, 451-452, the entire disclose of which is incorporated herein by reference
A composition is disclosed comprising
- at least one compound of formula (II) 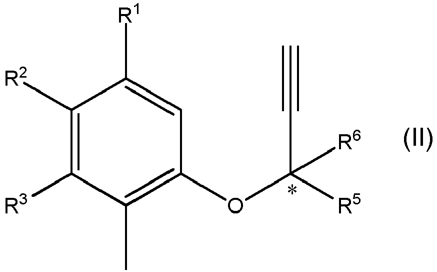
- and
   either
   at least one silver(I) or gold(I) salt or complex
   and
   at least one acid (**A**) which is selected from the group consisting acid (**A**) which is selected from the group consisting of BX₃, the adducts of BX₃ with alcohols or ethers, HBX₄, H[PX₆], H[SbF₆], HClO₄, H₂SO₄, trifluoroacetic acid, sulfonic acids and sulfonimides of formula (HAN-1); 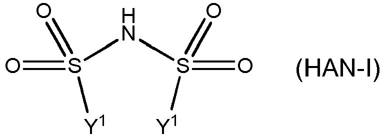 and/or
   at least one silver(I) or gold(I) metal salt or complex having an anion **(AN)** which is selected from the group consisting of [BX₄]⁻, [PX₆]⁻, [SbF₆]⁻, [ClO₄]⁻, CF₃COO⁻, sulfonates, tetra(3,5-bis(trifluoromethyl)phenyl)borate (BAr_{F}⁻), tetraphenylborate, and anions of formula (AN-1); 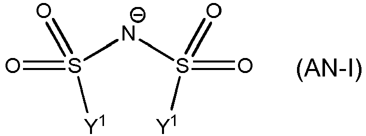 wherein X represents a halogen atom;
   and Y¹ represents a phenyl or a C₁₋₈-alkyl group which preferably is substituted by at least one halogen atom;
   wherein R¹ and R³ are independently from each other hydrogen or methyl groups;
   R² represents either hydrogen or a methyl group or a group OR^{2'} where R^{2'} represents hydrogen or a phenol protection group;
   R⁵ represents either a completely saturated C₁₋₂₅-alkyl group or a C₁₋₂₅-alkyl group comprising at least one carbon-carbon double bond;
   R⁶ represents either H or a C₁₋₂₅-alkyl group, particularly a methyl group; with the proviso that R⁵ is different from R⁶;
   and wherein each * represents a chiral centre.

Details of compound of formula (II), of silver(I) or gold(I) salt or complex, acid (**A**) and silver(I) or gold(I) metal salt or complex having an anion **(AN)** as well embodiments have already discussed above for the method of preparing compound of formula (I).

Compound of formula (I), particularly formula (I-A), preferably compound of formula (I-A-1), (I-A-1RR) or (I-A-2), are important intermediates for the synthesis of compounds of formula (IV).

Therefore, in a further aspect the present invention relates to a method of preparing compound of formula (IV) comprising
as described above the method of preparing compound of formula (I); followed by step d)
d) hydrogenation of compound of formula (I) in the presence of a hydrogenation catalyst

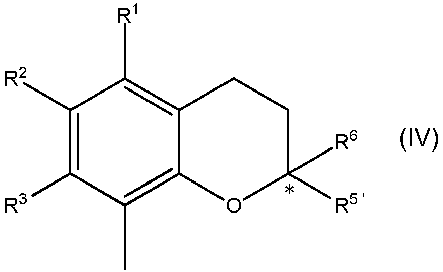

The groups R¹, R², R³ and R⁶ as well the preferred embodiments thereof have already discussed in details before for compound of formula (I) or (II). R^{5'} is R⁵ or the corresponding completely saturated C₁₋₂₅-alkyl group which results from hydrogenation of a group R⁵ which has at least one carbon-carbon double bond.

The chiral centre indicated by * of formula (IV) has the same configuration as in formula (I).

Preferred as compounds of formula (IV) are compounds of formula (IV-A) or (IV-ARR) or (IV-B)

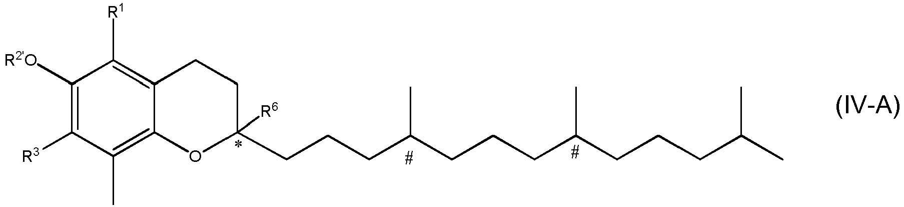

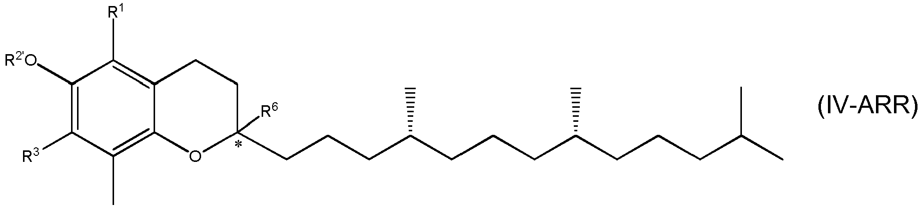

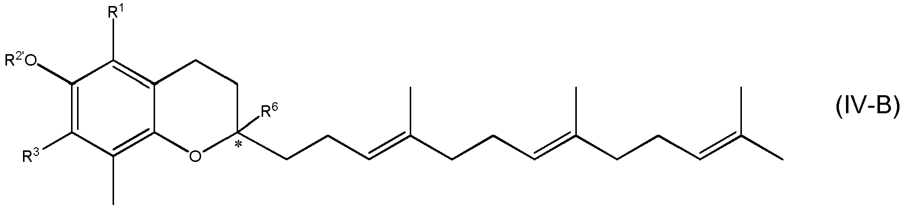

More preferred as compounds of formula (IV) are compounds of formula (IV-A-RRR) or (IV-B-R) 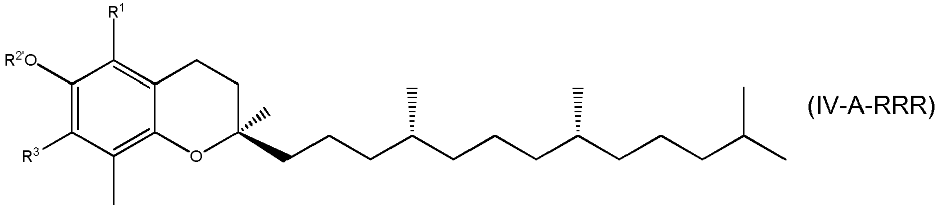 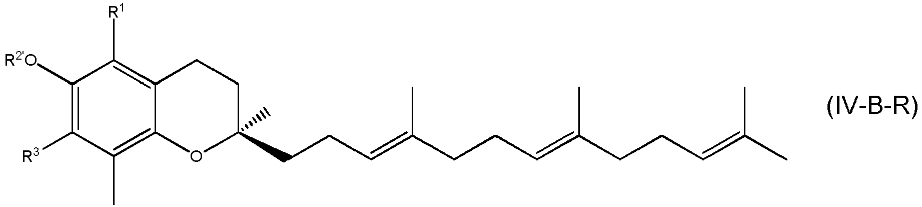 The group R^{2'} has been already discussed before in detail for formula (I) or (II).

The hydrogenation in step d) is made by molecular hydrogen in the presence of hydrogenation catalyst.

In case R⁵ represents in formula (I) a C₁₋₂₅-alkyl group comprising at least one carbon-carbon double bond, said side chain can be also hydrogenated. Therefore, in this case the R^{5'} represents in formula (IV) the corresponding hydrogenated R⁵ group from formula (I).

In case R⁵ represents a C₁₋₂₅-alkyl group comprising at least one carbon-carbon double bond depending on the hydrogenation the side chain can be hydrogenated in a stereospecific or non-stereo-specific way. In a non-stereo-specific way formation of racemic chiral centres is formed, i.e. mixtures having R and S configuration. In case the hydrogenation is stereospecific only a specific configuration, i.e. R or S is formed at the side chain.

Traditional hydrogenations such as disclosed in Kabbe and Heitzer, Synthesis 1978, 12,888-889 are non-stereo-specific hydrogenations. Asymmetric hydrogenations such as those using chiral iridium complexes as well as the method of asymmetrical hydrogenation of alkenes using chiral iridium complexes, preferably the one which is disclosed in WO 2006/066863 A1 or WO 2012/171969 A1, the entire content of which is hereby incorporated by reference, are stereospecific hydrogenations.

In case R⁵ represents an unsaturated C₁₋₂₅-alkyl group hydrogenation in step d) is preferably a stereo-specific hydrogenation is preferred.

In case R⁵ represents a saturated C₁₋₂₅-alkyl group hydrogenation in step d) is preferably a non-stereo-specific hydrogenation.

The present method leads in a high yield to the formation of formula (IV). It has been observed that the configuration is maintained during the reactions involved in the method. Hence, it is possible to have prepared compounds of formula (IV) in a high excess of a specific configuration at the chiral centre indicated by *. Particularly, it is possible to have prepared compounds of formula (IV) in a ratio of one configuration to the opposite configuration at the chiral carbon centre indicated by * of more than 90:10, preferably more than 95:5, particularly more than 98:2. Particularly it is possible to use this method for the production of tocopherols having the R-configuration at all chiral centres ((R,R,R)-tocopherols, R-tocotrienol), particularly (2R, 4'R, 8'R)-α-tocopherol or (2R, 4'R, 8'R)-α-tocopheryl acetate, or of (2R)-tocotrienols.

Furthermore disclosed is an use of a silver(I) or gold(I) salt or complex combined with at an acid (**A**) which is selected from the group consisting acid (**A**) selected from the group consisting of BX₃, the adducts of BX₃ with alcohols or ethers, HBX₄, H[PX₆], H[SbF₆], HClO₄, H₂SO₄, trifluoroacetic acid, sulfonic acids and sulfonimides of formula (HAN-1); 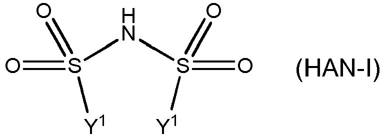
wherein X represents a halogen atom;
and Y¹ represents a phenyl or a C₁₋₈-alkyl group which preferably is substituted by at least one halogen atom;
for the intramolecular hydroarylation of chiral propargylic aryl ethers.

Details for this use have been already discussed in detail for the method of preparing compound of formula (I). Preferred chiral propargylic aryl ethers are compounds of formula (II).

Still further disclosed is an use of a silver(I) or gold(I) salt or complex having an anion (**AN**) which is selected from the group consisting of [BX₄]⁻, [PX₆]⁻, [SbF₆]⁻, [ClO₄]⁻, CF₃COO⁻, sulfonates, tetra(3,5-bis(trifluoromethyl)phenyl)borate (BAr_{F}⁻), tetraphenylborate, and anions of formula (AN-1); 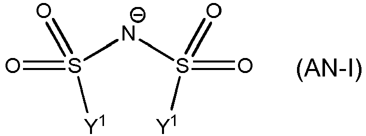
wherein X represents a halogen atom;
and Y¹ represents a phenyl or a C₁₋₈-alkyl group which preferably is substituted by at least one halogen atom;
for the intramolecular hydroarylation of chiral propargylic aryl ethers.

Details for this use have been already discussed in detail for the method of preparing compound of formula (I). Preferred chiral propargylic aryl ethers are compounds of formula (II).

In in the present invention no by-products having 5-membered rings or a different substitution pattern in view R¹, R² and R³ or a different position of the double bond or dimers have been observed, particularly also not in the presence of Ag(I) salts or complexes.

### Examples

The present invention is further illustrated by the following experiments.

### Analytical methods

### Gas Chromatography (GC):

The GC analyses have been performed on an Agilent HP-6850 series system using an FID-detector and helium gas as carrier. The separation is achieved on an HP-1 Methylsiloxane (30m x 0.32mm, 0.25 µm) column with the following temperature program 50°C (0 min) -> 10°/min -> 300°C (5 min). The samples were dissolved in ethyl acetate (5 mg in 1 mL) and 1 µL was injected with a split ratio of 50:1.

### HPLC:

The hydroarylation was monitored by HPLC on an Agilent 1200 series system using a YMC ProC18 column (150 x 4.6 mm), eluent acetonitrile : H₂O (7:3). Detection by DAD (=Diode Array Detector) at 210 nm & 230 nm.

### Chiral HPLC:

The diastereomeric (2R)- and (2S)-chromenes were monitored using a Chiralpak® OP(+) column, using 0.05% ethanol in hexane as eluent. Detection by DAD at 220 nm. The diastereomeric (3'R)- and (3'S)-aryl propargylic ethers were monitored using a Chiralpak® IA-3(+) column, using 0.05% ethanol in hexane as eluent. Detection by DAD at 220 nm. Chiralcel® OD-H columns were used for the analytical separation of dehydroisophytol and the corresponding carbonate (0.1% EtOH in hexane, detection: RI-detector, 1 mL/min).

### EXPERIMENTAL SERIES 1

In a first series of experiments the reactivity and conditions for the intramolecular hydroarylation have been studied on a basis of an achiral model molecule 2,3,6-trimethyl-4-((2-methylbut-3-yn-2-yl)oxy)phenyl acetate.

### Synthesis of methyl (2-methylbut-3-yn-2-yl)carbonate

5.0 g methylbutynol (MBI, 8, 59 mmol) were dissolved in 63 mL THF. Then 23.8 mL nBuLi (59 mmol, 2.5 M solution in hexane, 1 equiv) were added via syringe within 10 min keeping the inner temperature at about 0°C, resulting in a clear solution. Stirring was continued for 45 min at 0 °C. 4.7 mL methyl chloroformate (59 mmol, 1 equiv.) were added slowly via syringe keeping the inner temperature at about 0°C. After stirring at 0 °C the reaction mixture was then allowed to warm to 23 °C and stirred for 20 h. A white precipitate formed. The reaction mixture was diluted with 80 mL dichloromethane and quenched with 80 mL water. The aqueous layer was extracted with diethylether. The combined organic phases were washed with brine. The organic layer was dried over Na₂SO₄, filtered and concentrated at 20 °C bath temperature and 27 mbar for 1 h. 4.75 g of crude product, methyl (2-methylbut-3-yn-2-yl) carbonate, were obtained as a yellow oil with a purity of 59%. Purification was archived by distillation using bulb-to-bulb distillation at 64 °C and 10 mbar, furnishing 4.28 g (40% yield, purity 71 %) of methyl (2-methylbut-3-yn-2-yl) carbonate as a yellow oil.

### Characterization:

Boiling point: 64 °C at 10 mbar.
¹H NMR (CDCl₃, 300 MHz) δ 1.72 (s, 6H, 2 x CH₃), 2.57 (s, 1H, alkyne-H), 3.78 (3H, OCH₃) ppm.
¹³C NMR (CDCl₃, 300 MHz) δ 28.8 (2C), 54.4, 72.8, 73.8, 84.1, 153.5 ppm.

### Synthesis of 2,3,6-trimethyl-4-((2-methylbut-3-yn-2-yl)oxy)phenyl acetate

37.3g 4-hydroxy-2,3,6-trimethylphenyl acetate (DSM Nutritional Products, purity 99.9% GC, 192 mmol) were dissolved in 300 mL acetonitrile at -8°C. 28.6mg CuCl₂ (0.168 mmol, 0.1 mol%) were added, resulting in a yellow solution. 29.2g 1,8-Diazabicyclo[5.4.0]undec-7-ene (DBU) (192 mmol 1.1 equiv.) were added dropwise and the reaction mixture turned dark. Stirring was continued for 15 min at 0 °C. 24.4g methyl (2-methylbut-3-yn-2-yl) (172 mmol, 1 equiv.) as prepared above in 100 mL acetonitrile were added at 0 °C; the resulting mixture was stirred for 10 h at 0 °C. The reaction mixture was diluted with 1000 mL water and 1000 mL diethylether. The phases were separated and extracted with diethylether. The organic phase is washed with 40 mL of an aqueous solution of CuSO4 (10 wt%) followed by 60 mL brine. The combined organic phases were dried over Na₂SO₄, filtered and concentrated in vacuo (20 mbar at 45°C bath temperature). The crude product, 2,3,6-trimethyl-4-((2-methylbut-3-yn-2-yl)oxy)phenyl acetate, was obtained as yellow oil (37 g, purity 53%, quant. NMR). Purification by column chromatography (cyclohexane:EtOAc = 85:15) furnished 30.41 g (68% yield, purity of 99%) of 2,3,6-trimethyl-4-((2-methylbut-3-yn-2-yl)oxy)phenyl acetate as yellow oil.

### Characterization:

¹H NMR (CDCl₃, 300 MHz) δ 1.63 (s, 6H, C(CH₃)₂), 2.03 (s, 3H); 2.10 (s, 3H); 2.12 (s,3H), 2.32 (s, 3H); 2.52 (s, 1H, alkyne-H), 7.20 (s br, 1H, aryl-H) ppm.
¹³C NMR (CDCl₃, 300 MHz) δ 13.24, 13.31, 16.6, 20.6, 29.7 (2C), 72.6, 73.3, 86.7, 119.7, 126.8, 128.2, 129.5, 143.3, 151.2, 169.2 ppm.

### Synthesis of 2,2,5,7,8-pentamethyl-2H-chromen-6-yl acetate

2,3,6-trimethyl-4-((2-methylbut-3-yn-2-yl)oxy)phenyl acetate was added to the solvent indicated in table 1 to yield a 0.16 M solution in the propargylic aryl ether. Then the amount of silver (I) or gold(I) salt or complex and acid or at least one silver (I) or gold(I) metal salt or complex having an anion as indicated in table 1 are added in the amount indicated in table 1 and stirred at 25°C during 16 hours.

### Characterization:

¹H NMR (CDCl₃, 300 MHz) δ 1.40 (s, 6H, C(CH₃)₂), 2.02 (s, 3H, Ar-CH₃), 2.05 (s, 3H, Ar-CH₃), 2.10 (s, 3H, Ar-CH₃), 2.32 (s, 3H, CH₃CO₂), 6.47 (d, J = 10.0 Hz, 1H, =CH), 5.63 (d, J = 10.0 Hz, 1H, =CH) ppm.
¹³C NMR (CDCl₃, 300 MHz) δ 11.61, 11.64, 13.2, 20.6, 27.7, 46.0, 68.4, 75.0, 117.9, 119.6, 122.9, 129.0, 130.3, 141.4, 148.4, 169.5 ppm.

**Table 1. Results of hydroarylations of 2,3,6-trimethyl-4-((2-methylbut-3-yn-2-yl)oxy)phenyl acetate.**

| **Example** | catalyst | [mol-%]* | Acid | [mol-%]* | Solvent | Conversion⁴ [Wt%]³ | Yield⁵ [Wt%]³ |
|---|---|---|---|---|---|---|---|
| ***Ref.1*** | Ph₃PAuCl | 0.3 | | | CH₂Cl₂ | 0 | 0 |
| ***Ref.2*** | Ph₃PAuCl | 0.1 | | | toluene | 0 | 0 |
| ***Ref.3*** | | | HBF₄ | 0.1 | toluene | 5 | 0 |
| ***Ref.4*** | | | TfOH¹ | 0.1 | toluene | 0 | 0 |
| ***Ref.5*** | | | TfOH¹ | 0.1 | CH₂Cl₂ | 0 | 0 |
| ***Comp.1*** | Ph₃PAuCl | 0.1 | BF₃•OEt₂ | 0.5 | CH₂Cl₂ | 100 | >99 |
| ***Comp.2*** | Ph₃PAuCl | 0.6 | BF₃•OEt₂ | 0.5 | toluene | 100 | >99 |
| ***Comp.3*** | Ph₃PAuCl | 0.1 | HBF₄ | 0.1 | CH₂Cl₂ | 100 | 83 |
| ***Comp.4*** | Ph₃PAuCl | 0.1 | HBF₄ | 0.1 | toluene | 100 | 87 |
| ***Comp.5*** | AgBF₄ | 1.2 | | | toluene | 33 | 26 |
| ***Comp.6*** | AgBF₄ | 1.8 | | | toluene | 97 | 99 |
| ***Comp.7*** | AgOTf² | 1.8 | | | CH₂Cl₂ | 100 | 99 |
| ***Comp.8*** | AgOTf² | 0.6 | | | toluene | 89 | 88 |

| | | | | | | | |
|---|---|---|---|---|---|---|---|
| *relative to 2,3,6-trimethyl-4-((2-methylbut-3-yn-2-yl)oxy)phenyl acetate ¹TfOH = triflic acid ²AgOTf = silver triflate ³wt% = % by weight ⁴conversion has been determined by quantitative LC and is indicated relative to 2,3,6-trimethyl-4-((2-methylbut-3-yn-2-yl)oxy)phenyl acetate. ⁵yield has been determined by quantitative LC and is indicated relative to 2,3,6-trimethyl-4-((2-methylbut-3-yn-2-yl)oxy)phenyl acetate. | | | | | | | |

### EXPERIMENTAL SERIES 2

In a second series of experiments the learning of the first experimental series has been applied to chiral compounds.

### Synthesis of methyl (3,7,11-trimethyldodec-1-yn-3-yl) carbonate

5.35 g all-racemic dehydroisophytol (14.0 mmol) was dissolved in 60 mL THF. Then 8.7 mL nBuLi (2.5M; 21.8 mmol; 1.2 equiv.) were added via syringe within 15 min keeping the temperature at about 0°C. A yellowish solution formed. After complete addition, stirring was continued at -2 °C for 90 min. 2.13 mL methyl chloroformate (27.2 mmol, 1.5 equiv.) was added via syringe. The mixture was allowed to slowly warm to 23 °C overnight. A white suspension formed in a yellow liquid phase. The mixture was quenched with 100 mL HCI (1N) and with diethylether. The combined organic layers are washed with brine, dried over MgSO₄ and concentrated in vacuo (12 mbar at 35°C) over 1 h. 5.53 g of crude product, methyl (3,7,11-trimethyldodec-1-yn-3-yl) carbonate, was obtained as a yellow oil with a purity of 69% (determined by NMR). The crude product was purified over column chromatography (heptane/AcOEt, 9/1, Rf=0.4) and methyl (3,7,11-trimethyldodec-1-yn-3-yl) carbonate has been isolated.

### Characterization:

¹H NMR (CDCl₃, 300 MHz) δ 0.83 (s, 3H); 0.86 (s, 6 H); 0.88 (s, 3H) 1.07-1.40 (m, 17H); 1.50-1.57 (m, 2H); 1.71 (s, 3H); 1.86 (m, 2H); 2.58 (s, 1H); 3.77 (s,3H) ppm. ¹³C NMR (CDCl₃, 300 MHz) δ 19.55; 19.69; 21.58; 22.65; 22.74; 24.47; 24.81;24.83; 26.28; 28.00; 32.65; 32.68; 32.79; 32.81; 36.81; 36.90; 37.30; 37.37; 37.41;39.39; 41.63; 54.34; 73.73; 83.34; 153.58 ppm.

### Synthesis of 2,3,6-trimethyl-4-((3,7,11,15-tetramethylhexadec-1-yn-3-yl)oxy)phenyl acetate

1.50 g 4-hydroxy-2,3,6-trimethylphenyl acetate (DSM Nutritional Products, purity 99.9% GC, 7.7 mmol) are dissolved in 60 mL acetonitrile and cooled to 0°C. Then 41 mg copper(II) chloride (0.31 mmol, 0.039 equiv.) are added and 1.59 mL 1,8-Diazabicyclo[5.4.0]undec-7-ene (DBU) (10.6 mmol, 1.4 equiv.) are added dropwise. The reaction mixture turned dark. Stirred was continued for 15 minutes at 1°C. Then 2.96 g methyl (3,7,11-trimethyldodec-1-yn-3-yl) carbonate as prepared above (7.7 mmol, 1 equiv) dissolved in 8 mL acetonitrile were added at 1°C and then stirred for 3.5 hours at 0°C. The reaction was followed by TLC. After 3.5 h no carbonate was detected anymore. The reaction mixture is evaporated at 16 mbar and 35°C bath temperature. Then 40 mL water and 50 mL diethylether have been added leading to a slight emulsion. The phases are separated and the aqueous phase is extracted with diethylether. The organic phase is then washed with 160 mL CuSO₄ 10% aqueous solution and 40 mL brine and then dried over Na₂SO₄ and concentrated at 40°C und 10 mbar. 4.4 g of a brown oil are obtained containing the product in 30% purity (quant. NMR). The aryl propargylether was purified by column chromatography (SiO₂) using heptane and diethylether as eluents (heptane to diethylether = 97:3). The product 2,3,6-trimethyl-4-((3,7,11,15-tetramethylhexadec-1-yn-3-yl)oxy)phenyl acetate was obtained in 19% yield.

### Characterization:

¹H NMR (CDCl₃, 300 MHz) δ 0.84-0.89 (m, 12H), 1.02-1.45 (m, 16H), 1.54 (s, 3H, Cq(CH₃)), 1.57-1.95 (m, 5H), 2.03 (s, 3H, Ar-CH₃), 2.10 (s, 3H, Ar-CH₃), 2.11 (s, 3H, Ar-CH₃), 2.32 (s, 3H, CH₃CO₂), 2.53 (s, 1H, alkyne-H), 7.20 (s, 1H, Ar-H) ppm. ¹³C NMR (CDCl₃, 300 MHz) δ 13.2, 16.6, 19.6, 19.7, 19.8, 20.6, 21.9, 22.7, 22.8, 24.5, 24.8, 26.8, 28.0, 32.7, 32.8, 37.1, 37.2, 37.3, 37.4, 37.5, 39.4, 43.3, 68.4, 74.5, 85.9, 119.4, 126.6, 128.0, 129.4, 143.1, 151.2, 169.2 ppm.

### Synthesis of 2,5,7,8-tetramethyl-2-(4,8,12-trimethyltridecyl)-2H-chromen-6-yl acetate

2,3,6-trimethyl-4-((3,7,11,15-tetramethylhexadec-1 -yn-3-yl)oxy)phenyl acetate was added to the solvent indicated in table 2 to yield a 0.16 M solution in the propargylic aryl ether. Then the amount of silver(I) or gold(I) salt or complex and acid or at least one silver(I) or gold(I) metal salt or complex having an anion as indicated in table 2 are added in the amount indicated in table 2 and stirred at 25°C during the time indicated in table 2.

### Characterization:

¹H NMR (CDCl₃, 300 MHz) δ 0.86 (s, 3 H), 0.89 (s, 6 H) 0.91 (s, 3 H) 1.24 - 1.61 (m, 24 H) 2.05 (s, 3 H) 2.08 (s, 3 H) 2.13 (s, 3 H) 2.35 (s, 3 H) 5.62 (d, J=10.17 Hz, 1 H) 6.52 (d, J=10.17 Hz, 1 H) ppm.
¹³C NMR (CDCl₃, 300 MHz) δ 11.6 (2C), 13.2, 19.7, 19.8, 20.6, 21.5, 22.4, 22.7, 22.8, 24.5, 24.9, 25.9, 28.0, 32.7, 32.8, 37.3, 37.4, 39.4, 41.1, 77.4, 117.7, 119.8, 122.4, 122.7, 129.0, 129.7, 141.3, 148.5, 169.5 ppm.

**Table 2. Results of hydroarylations of 2,3,6-trimethyl-4-((3,7,11,15-tetramethylhexadec-1-yn-3-yl)oxy)phenyl acetate.**

| **Example** | catalyst | [mol-%]/* | Co-catalyst | [mol-%]* | Solvent | Time [h] | Conversion⁴ [Wt%]³ | Yield⁵ [wt%]3 |
|---|---|---|---|---|---|---|---|---|
| ***Ref.6*** | Ph₃PAuCl | 3 | | | toluene | 16 | 0 | 0 |
| ***1*** | Ph₃PAuCl | 10 | BF₃•OEt₂ | 10 | toluene | 16 | 100 | 81 |
| ***2*** | Ph₃PAuCl | 3 | BF₃•OEt₂ | 0.5 | toluene | 10 | 85 | 72 |
| ***3*** | Ph₃PAuCl | 3 | BF₃•OEt₂ | 3 | toluene | 16 | 100 | 81 |
| ***4*** | AgOTf² | 10 | | | toluene | 16 | 61 | 40 |
| ***5*** | Et₃PAuCl | 3 | BF₃•OEt₂ | 3 | toluene | 16 | 68 | 59 |
| ***6*** | Et₃PAuCl | 3 | BF₃•OEt₂ | 3 | nitromethane | 2.5 | 92 | 74 |
| ***7*** | Et₃PAuCl | 3 | BF₃•OEt₂ | 3 | toluene | 17 | 68 | 59 |
| ***8*** | Ph₃PAuNTf₂ | 1 | | | CH₂Cl₂ | 1.3 | 97 | 94 |
| ***9*** | Ph₃PAuNTf₂ | 0.2 | | | CH₂Cl₂ | 2 | 99 | 85 |
| ***10*** | JohnPhosAuSbF₆I | 1 | | | CH₂Cl₂ | 3.5 | 98 | 89 |
| ***11*** | [(iPr₂)Ph₂]ImAuCl | 1 | AgSbF₆ | 1 | CH₂Cl₂ | 24 | 43 | 28 |

| | | | | | | | | |
|---|---|---|---|---|---|---|---|---|
| ^{*}relative to 2,3,6-trimethyl-4-((3,7,11,15-tetramethylhexadec-1-yn-3-yl)oxy)phenyl acetate ²AgOTf = silver triflate ³wt% = % by weight ⁴conversion has been determined by quantitative LC and is indicated relative to 2,3,6-trimethyl-4-((3,7,11,15-tetramethylhexadec-1-yn-3-yl)oxy)phenyl acetate. ⁵Yield has been determined by quantitative NMR and is indicated relative to 2,3,6-trimethyl-4-((3,7,11,15-tetramethylhexadec-1-yn-3-yl)oxy)phenyl acetate. | | | | | | | | |

### EXPERIMENTAL SERIES 3

In a third series of experiments the process was applied to chiral compounds in isomerically pure form

### Synthesis of methyl ((3R,7R,11R)-3,7,11,15-tetramethylhexadec-1-yn-3-yl) carbonate

The synthesis of ((3R,7R,1 1 R)-3,7,1 1,15-tetramethylhexadec-1-yn-3-yl) carbonate was identical to the synthesis of methyl (3,7,11-trimethyldodec-1-yn-3-yl) carbonate as described above in experimental series 2 except that RRR-dehydroisophytol ((3R,7R,11R)-3,7,1,15-tetramethylhexadec-1-yn-3-ol), which has been synthesized from natural (E,R,R)-phytol being epoxidized by Sharpless oxidation, converted to the corresponding epoxychloride, followed by multiple lithiation as disclosed by Takano S. et al in Synlett 1990(8), 451-452.

### Synthesis of (R)-2,5,7,8-tetramethyl-2-((4R,8R)-4,8,12-trimethyltridecyl)-2H-chromen-6-yl acetate

The synthesis of (R)-2,5,7,8-tetramethyl-2-((4R,8R)-4,8,12-trimethyltridecyl)-2H-chromen-6-yl acetate is identical to the synthesis of 2,5,7,8-tetramethyl-2-(4,8,12-trimethyltridecyl)-2H-chromen-6-yl acetate as described above in experimental series 2 except that ((3R,7R,11R)-3,7,11,15-tetramethylhexadec-1-yn-3-yl) carbonate was used as starting product.

### EXPERIMENTAL SERIES 4

In a forth series of experiments the process was applied to chiral compounds in isomerically pure form.

2,3,6-trimethyl-4-((3,7,11,15-tetramethylhexadec-1 -yn-3-yl)oxy)phenyl acetate as prepared above in experimental series 2 is an all-rac mixture.

It has been separated by preparative HPLC using a chiral stationary phase (Chiralpak® IA-3) into two fractions. The first fraction (FR1) consists primarily of 2,3,6-trimethyl-4-(((3R,7SR,11SR)-3,7,11,15-tetramethylhexadec-1-yn-3-yl)oxy)phenyl acetate ("*3R,7SR,11SR"*)*.* The second fraction (FR2) consists primarily of 2,3,6-trimethyl-4-(((3S,7SR,11SR)-3,7,11,15-tetramethylhexadec-1-yn-3-yl)oxy)phenyl acetate ("*3S,7SR,11SR*"). Therefore, the chromatography using a chiral stationary phase resulted in a separation of the isomers having different configuration at the chiral carbon centre next to the ether oxygen atom.

**Table 3 Amounts of isomers in fraction 1 and fraction 2.**

| | Amounts in fraction 1 (FR1) [%] | Amounts in fraction 2 (FR2) [%] |
|---|---|---|
| *3S,7SR,11SR* | 94 | 20 |
| *3R,7SR,11SR* | 6 | 80 |

### Synthesis of (R)-2,5,7,8-tetramethyl-2-((4SR,8SR)-4,8,12-trimethyltridecyl)-2H-chromen-6-yl acetate and (R)-2,5,7,8-tetramethyl-2-((4SR,8SR)-4,8,12-trimethyltridecyl)-2H-chromen-6-yl acetate

Fraction 1, respectively fraction 2, as separated above, was added in example ***12,*** resp. example ***13,*** to toluene to yield a 0.16 M solution in the propargylic aryl ether. Then 3 mol % of Ph₃PAuCl and 3 mol-% of BF₃•OEt₂ have been added and stirred at 25°C during 20 hours.

The product has been analysed by chiral HPLC and the amounts of (R)-2,5,7,8-tetramethyl-2-((4SR,8SR)-4,8,12-trimethyltridecyl)-2H-chromen-6-yl acetate (*"R,4SR,8SR*") and (S)-2,5,7,8-tetramethyl-2-((4SR,8SR)-4,8,12-trimethyltridecyl)-2H-chromen-6-yl acetate (*"S,4SR,8SR*) have been indicated in table 4.

**Table 4 Amounts of isomers starting from fraction 1 or fraction 2.**

| | ***12*** | **13** |
|---|---|---|
| | (using fraction 1 (FR1) [%] | (using fraction 2 (FR2) [%] |
| *S,4SR,8SR* | 93 | 21 |
| *R,4SR,8SR* | 7 | 79 |

The experiments from table 4 and table 3 prove that the cyclisation is done under retension of the configuration.

### Synthesis of α-tocopheryl acetate

2,5,7,8-tetramethyl-2-(4,8,12-trimethyltridecyl)-2H-chromen-6-yl acetate, (R)-2,5,7,8-tetramethyl-2-((4R,8R)-4,8,12-trimethyltridecyl)-2H-chromen-6-yl acetate, (R)-2,5,7,8-tetramethyl-2-((4SR,8SR)-4,8,12-trimethyltridecyl)-2H-chromen-6-yl acetate and (S)-2,5,7,8-tetramethyl-2-((4SR,8SR)-4,8,12-trimethyltridecyl)-2H-chromen-6-yl acetate of series 2, 3 or 4, respectively, have been hydrogenated according the procedure disclosed by Kabbe and Heitzer, Synthesis 1978; 12, 888-889. The hydrogenation yielded in all cases α-tocopheryl acetate. The identity of α-tocopheryl acetate and the retention of the configuration and ratios at the chiral carbon centres at the 2-position as well as the 4' and 8' positions could be estabilished by chromatographic analysis.

## Claims

1. Method of preparing compound of formula (I) comprising the steps 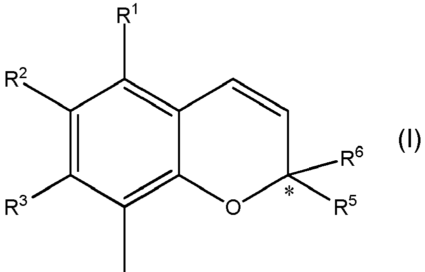
a) providing compound of formula (II) 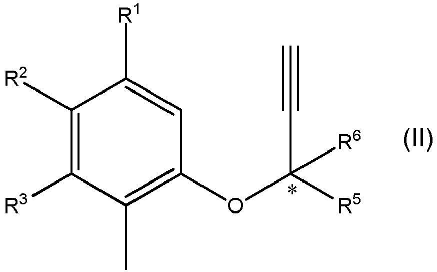
b) adding
at least one silver(I) or gold(I) salt or complex and
at least one acid (**A**) which is selected from the group consisting of BX₃, the adducts of BX₃ with alcohols or ethers, HBX₄, H[PX₆], H[SbF₆], HClO₄, H₂SO₄, trifluoroacetic acid, sulfonic acids and sulfonimides of formula (HAN-1); 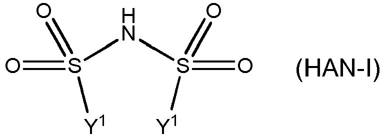 and/or
at least one silver(I) or gold(I) metal salt or complex having an anion (**AN**) which is selected from the group consisting of [BX₄]⁻, [PX₆]⁻, [SbF₆]⁻, [ClO₄]⁻, CF₃COO⁻, sulfonates, tetra(3,5-bis(trifluoromethyl)phenyl)borate (BAr_{F}⁻), tetraphenylborate, and anions of formula (AN-1); 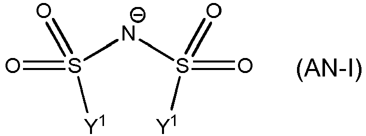 wherein X represents a halogen atom;
and Y¹ represents a phenyl or a C₁₋₈-alkyl group which preferably is substituted by at least one halogen atom
to compound of formula (II) forming a mixture;
c) submitting said mixture to a temperature in the range between -15°C and 40°C, preferably between 15°C and 40°C;
wherein R¹ and R³ are independently from each other hydrogen or methyl groups;
R² represents either hydrogen or a methyl group or a group OR^{2'} where R^{2'} represents hydrogen or a phenol protection group;
R⁵ represents either completely saturated C₁₋₂₅-alkyl group or a C₁₋₂₅-alkyl group comprising at least one carbon-carbon double bond;
R⁶ represents either H or a C₁₋₂₅-alkyl group, particularly a methyl group; with the proviso that R⁵ is different from R⁶;
and wherein each * represents a chiral centre and this chiral centre has the same configuration in formula (I) and formula (II)
and wherein the compound of formula (II) is obtained by the reaction

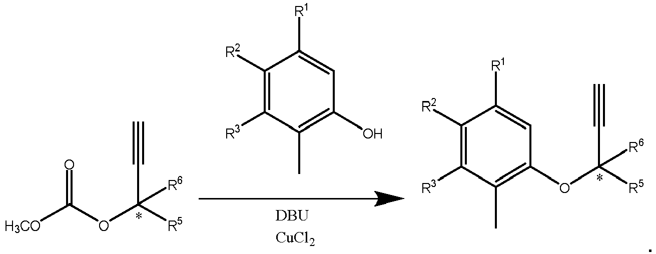

2. Method according to claim 1, **characterized in that** R⁵ is of formula (III) 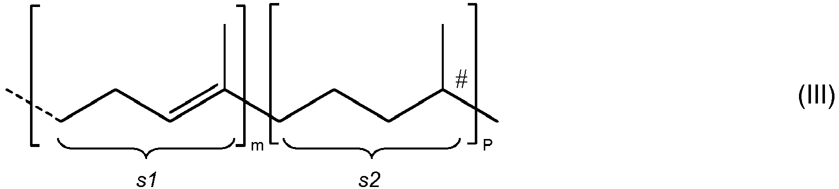 wherein m and p stand independently from each other for a value of 0 to 5 provided that the sum of m and p is 1 to 5,
and where the substructures in formula (III) represented by *s1* and *s2* can be in any sequence;
and the dotted line represents the bond by which the substituent of formula (III) is bound to the rest of the compound of formula (II-B) or formula (I);
and wherein # represents a chiral centre, obviously except in case where said centre is linked to two methyl groups.

3. Method according to claim 1 or claim 2, **characterized in that** the acid (**A**) is BX₃, particularly BF₃, respectively the adducts thereof with alcohols or ethers, particularly BF₃-dialkylether adducts.

4. Method according to anyone of the preceding claims **characterized in that** the acid (**A**) in step b) is an acid which is selected from the group consisting of BF₃, HBF₄, triflic acid and triflimidic acid.

5. Method according to anyone of the preceding claims **characterized in that** that the anion (**AN**) in step b) is an anion which is selected from the group consisting of [BX₄]⁻, triflate, and anions of formula (AN-1).

6. Method according to anyone of the preceding claims **characterized in that** in step b) the silver(I) or gold(I) salt or complex is a chloro complex of Au(I) and the acid (**A**) is BF₃, respectively the adducts thereof with alcohols or ethers, particularly BF₃-dialkylether adducts.

7. Method according to anyone of the preceding claims **characterized in that** in step b) the silver(I) or gold(I) salt or complex having an anion (**AN**) is silver(I) triflate or silver(I) tetrafluoroborate.

8. Method according to anyone of the preceding claims **characterized in that** in step b) a combination of Ag(I) triflate or Ag(I) tetrafluoroborate and a chloro complex of Au(I) is used.

9. Method of preparing compound of formula (IV) comprising the method according to anyone of the claims 1-8; followed by step d)
d) hydrogenation of compound of formula (I) in the presence of a hydrogenation catalyst 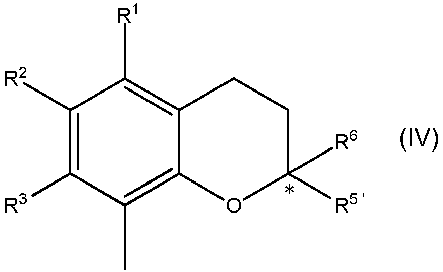 wherein
R^{5'} is R⁵ or the corresponding completely saturated C₁₋₂₅-alkyl group; and
as the chiral centre indicated by * of formula (IV) has the same configuration as in formula (I).

## Patentansprüche

1. Verfahren zur Herstellung einer Verbindung der Formel (I), mit den Schritten 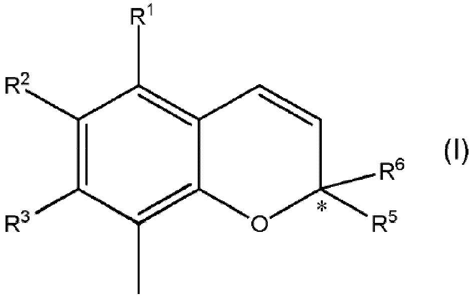
a) Bereitstellen einer Verbindung der Formel (II) 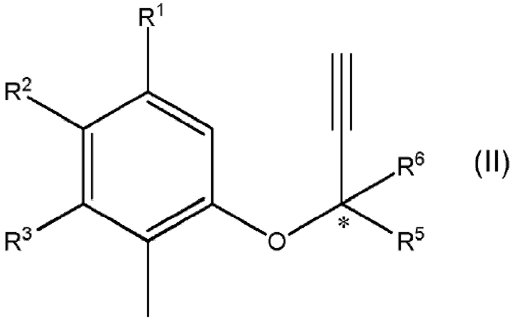
b) Zugeben von
mindestens einem Silber(I)- oder Gold(I)-Salz oder -Komplex und
mindestens einer Säure (**A**), die aus der Gruppe bestehend aus BX₃, den Addukten von BX₃ mit Alkoholen oder Ethern, HBX₄, H[PX₆], H[SbF₆], HClO₄, H₂SO₄, Trifluoressigsäure, Sulfonsäuren und Sulfonimiden der Formel (HAN-1) ausgewählt ist; 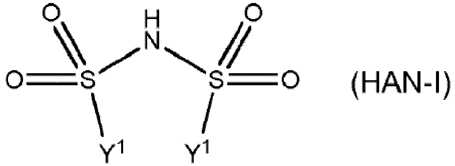 und/oder
mindestens einem Silber(I)- oder Gold(I)-Salz oder -Komplex mit einem Anion (**AN**), das aus der Gruppe bestehend aus [BX₄]⁻, [PX₆]⁻, [SbF₆]⁻, [ClO₄]⁻, CF₃COO⁻, Sulfonaten, Tetra-(3,5-bis(trifluormethyl)phenyl)borat (BAr_{F}⁻), Tetraphenylborat und Anionen der Formel AN-1 ausgewählt ist; 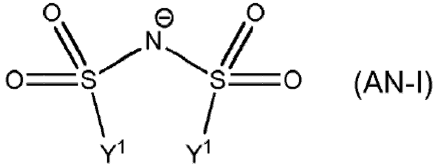 wobei X für ein Halogenatom steht und Y¹ für eine Phenyl- oder C₁-C₈-Alkylgruppe, die vorzugsweise durch mindestens ein Halogenatom substituiert ist, steht,
zu einer Verbindung der Formel (II) unter Bildung einer Mischung;
c) Einwirkenlassen einer Temperatur im Bereich zwischen -15 °C und 40 °C, vorzugsweise zwischen 15 °C und 40 °C, auf die Mischung;
wobei R¹ und R³ unabhängig voneinander für Wasserstoff oder Methylgruppen stehen;
R² für Wasserstoff oder eine Methylgruppe oder eine Gruppe OR^{2'} steht, wobei R^{2'} für Wasserstoff oder eine Phenol-Schutzgruppe steht;
R⁵ für eine vollständig gesättigte C₁₋₂₅-Alkylgruppe oder eine C₁₋₂₅-Alkylgruppe mit mindestens einer Kohlenstoff-Kohlenstoff-Doppelbindung steht;
R⁶ für H oder eine C₁₋₂₅-Alkylgruppe, insbesondere eine Methylgruppe, steht; mit der Maßgabe, dass R⁵ von R⁶ verschieden ist;
und wobei jedes * für ein Chiralitätszentrum steht und dieses Chiralitätszentrum in Formel (I) und Formel (II) die gleiche Konfiguration aufweist und wobei die Verbindung der Formel (II) durch die Umsetzung 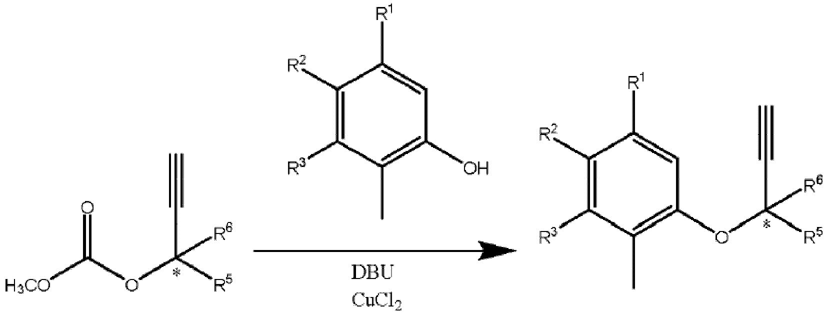 erhalten wird.

2. Verfahren nach Anspruch 1, **dadurch gekennzeichnet, dass** R⁵ die Formel (III) aufweist: 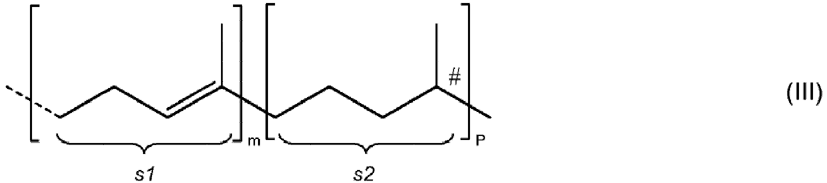 wobei m und p unabhängig voneinander für einen Wert von 0 bis 5 stehen, mit der Maßgabe, dass die Summe von m und p 1 bis 5 beträgt,
und wobei die durch *s1* und *s2* wiedergegebenen Unterstrukturen in Formel (III) in beliebiger Reihenfolge vorliegen können;
und die gestrichelte Linie für die Bindung steht, über die der Substituent der Formel (III) an den Rest der Verbindung der Formel (II-B) oder Formel (I) gebunden ist;
und wobei # für ein Chiralitätszentrum steht, offensichtlich mit Ausnahme des Falls, in dem das Zentrum an zwei Methylgruppen gebunden ist.

3. Verfahren nach Anspruch 1 oder Anspruch 2, **dadurch gekennzeichnet, dass** es sich bei der Säure (**A**) um BX₃, insbesondere BF₃, bzw. die Addukte davon mit Alkoholen oder Ethern, insbesondere BF₃-Dialkylether-Addukte, handelt.

4. Verfahren nach einem der vorhergehenden Ansprüche, **dadurch gekennzeichnet, dass** es sich bei der Säure (**A**) in Schritt b) um eine Säure handelt, die aus der Gruppe bestehend aus BF₃, HBF₄, Trifluormethansulfonsäure und Bis(trifluormethan)sulfonimid ausgewählt ist.

5. Verfahren nach einem der vorhergehenden Ansprüche, **dadurch gekennzeichnet, dass** es sich bei dem Anion (**AN**) in Schritt b) um ein Anion handelt, das aus der Gruppe bestehend aus [BX₄]⁻, Triflat und Anionen der Formel (AN-1) ausgewählt ist.

6. Verfahren nach einem der vorhergehenden Ansprüche, **dadurch gekennzeichnet, dass** es sich in Schritt b) bei dem Silber(I)- oder Gold(I)-Salz oder -Komplex um einen Chlorokomplex von Au(I) handelt und es sich bei der Säure (**A**) um BF₃ bzw. die Addukte davon mit Alkoholen oder Ethern, insbesondere BF₃-Dialkylether-Addukte, handelt.

7. Verfahren nach einem der vorhergehenden Ansprüche, **dadurch gekennzeichnet, dass** es sich in Schritt b) bei dem Silber(I)- oder Gold(I)-Salz oder -Komplex mit einem Anion (**AN**) um Silber(I)-triflat oder Silber(I)-tetrafluoroborat handelt.

8. Verfahren nach einem der vorhergehenden Ansprüche, **dadurch gekennzeichnet, dass** in Schritt b) eine Kombination von Ag(I)-Triflat oder Ag(I)-Tetrafluoroborat und einem Chlorokomplex von Au(I) verwendet wird.

9. Verfahren zur Herstellung einer Verbindung der Formel (IV), umfassend
das Verfahren nach einem der Ansprüche 1-8; gefolgt von Schritt d)
d) Hydrierung einer Verbindung der Formel (I) in Gegenwart eines Hydrierkatalysators 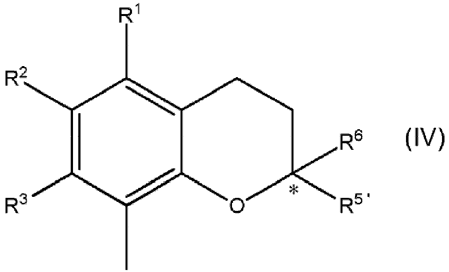 wobei
R^{5'} für R⁵ oder die entsprechende vollständig gesättigte C₁₋₂₅-Alkylgruppe steht und
das durch * angezeigte Chiralitätszentrum der Formel (IV) die gleiche Konfiguration wie in Formel (I) aufweist.

## Revendications

1. Procédé de préparation d'un composé de formule (I) comprenant les étapes suivantes 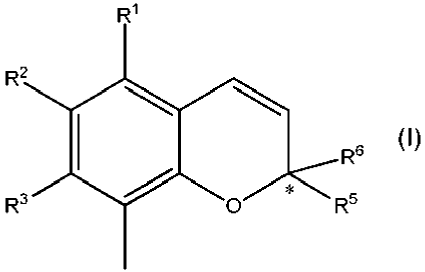
a) fourniture d'un composé de formule (II) 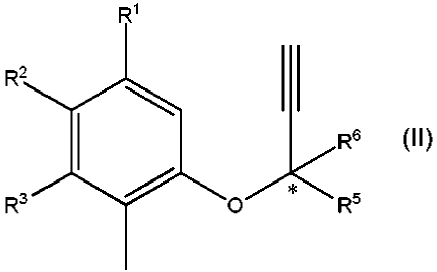
b) ajout de
au moins un sel ou complexe d'argent (I) ou d'or (I) et au moins un acide (A) qui est choisi dans le groupe constitué de BX₃, d'adduits de BX₃ avec des alcools ou des éthers, HBX₄, H[PX₆], H[SbF₆], HClO₄, H₂SO₄, de l'acide trifluoroacétique, d'acides sulfoniques et de sulfonimides de formule (HAN-1) ; 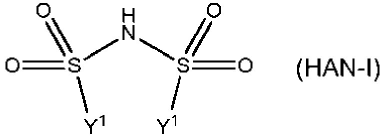 et/ou
au moins un sel ou complexe de métal d'argent (I) ou d'or (I) ayant un anion (AN) qui est choisi dans le groupe constitué de [BX₄]⁻, [PX₆]⁻, [SbF₆]⁻, [ClO₄]⁻, CF₃COO⁻, sulfonates, tétra(3,5-bis(trifluoro-méthyl)phényl)borate (BAr_{F}⁻), tétraphénylborate, et d'anions de formule (AN-1) ; 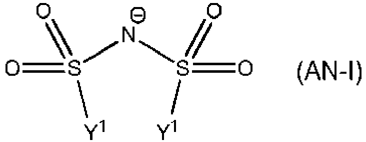 dans lequel X représente un atome d'halogène ;
et Y¹ représente un groupe phényle ou alkyle en C₁₋₈ qui est de préférence substitué par au moins un atome d'halogène
à un composé de formule (II) pour former un mélange ;
c) soumission dudit mélange à une température dans la plage comprise entre -15 °C et 40 °C, de préférence entre 15 °C et 40 °C ;
dans lequel R¹ et R³ sont, indépendamment l'un de l'autre, hydrogène ou des groupes méthyle ;
R² représente hydrogène ou un groupe méthyle ou un groupe OR^{2'} où R^{2'} représente hydrogène ou un groupe protecteur de phénol ;
R⁵ représente un groupe alkyle en C₁₋₂₅ complètement saturé ou un groupe alkyle en C₁₋₂₅ comprenant au moins une double liaison carbone-carbone ;
R⁶ représente H ou un groupe alkyle en C₁₋₂₅, en particulier un groupe méthyle ; à condition que R⁵ soit différent de R⁶ ;
et dans lequel chaque * représente un centre chiral et ce centre chiral a la même configuration dans la formule (I) et la formule (II)
et dans lequel le composé de formule (II) est obtenu par la réaction

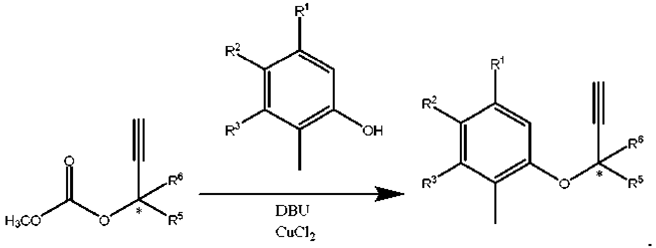

2. Procédé selon la revendication 1, **caractérisé en ce que** R⁵ est de formule (III) 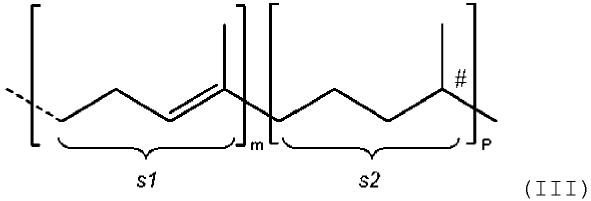 dans laquelle m et p désignent, indépendamment l'un de l'autre, une valeur de 0 à 5 à condition que la somme de m et p soit de 1 à 5,
et où les sous-structures dans la formule (III) représentées par *s1* et *s2* peuvent être dans une séquence quelconque ;
et le trait pointillé représente la liaison par laquelle le substituant de formule (III) est lié au reste du composé de formule (II-B) ou de la formule (I) ;
et dans lequel # représente un centre chiral, évidemment à l'exception du cas dans lequel ledit centre est lié à deux groupes méthyle.

3. Procédé selon la revendication 1 ou la revendication 2, **caractérisé en ce que** l'acide (A) est BX₃, en particulier BF₃, respectivement les adduits de celui-ci avec des alcools ou des éthers, en particulier des adduits BF₃-éther de dialkyle.

4. Procédé selon l'une quelconque des revendications précédentes, **caractérisé en ce que** l'acide (A) dans l'étape b) est un acide qui est choisi dans le groupe constitué de BF₃, HBF₄, l'acide triflique et l'acide triflimidique.

5. Procédé selon l'une quelconque des revendications précédentes **caractérisé en ce que** l'anion (AN) dans l'étape b) est un anion qui est choisi dans le groupe constitué de [BX₄]⁻, du triflate, et des anions de formule (AN-1).

6. Procédé selon l'une quelconque des revendications précédentes, **caractérisé en ce que**, dans l'étape b), le sel ou complexe d'argent (I) ou d'or (I) est un complexe de chloro d'Au(I) et l'acide (A) est BF₃, respectivement les adduits de ceux-ci avec des alcools ou des éthers, en particulier des adduits BF₃-éther de dialkyle.

7. Procédé selon l'une quelconque des revendications précédentes, **caractérisé en ce que**, dans l'étape b), le sel ou complexe d'argent (I) ou d'or (I) ayant un anion (AN) est le triflate d'argent (I) ou le tétrafluoroborate d'argent (I).

8. Procédé selon l'une quelconque des revendications précédentes, **caractérisé en ce que**, dans l'étape b), une combinaison de triflate d'Ag(I) ou de tétrafluoroborate d'Ag(I) et d'un complexe de chloro d'Au(I) est utilisé.

9. Procédé de préparation d'un composé de formule (IV) comprenant le procédé selon l'une quelconque des revendications 1 à 8 ; suivi de l'étape d) d) hydrogénation du composé de formule (I) en présence d'un catalyseur d'hydrogénation 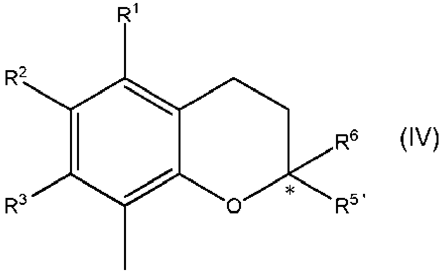 dans lequel
R^{5'} est R⁵ ou le groupe alkyle en C₁₋₂₅ complètement saturé correspondant ; et en tant que centre chiral indiqué par * de formule (IV), a la même configuration que dans la formule (I).
